# EUROPEAN PATENT APPLICATION

(11) **EP 4 578 468 A1**
(43) Date of publication of application: **02.07.2025**
(21) Application number: 24841411.2
(22) Date of filing: 29.08.2024
(51) Int. Cl.: A61L 9/03

(54) **FRAGRANCE DEVICE**

(30) Priority: 17.11.2023 CN 202323122105 U; 17.11.2023 CN 202311545636
(71) Applicant: Shenzhen Fenyue Technology Co., Ltd, Shenzhen, Guangdong 518101 (CN)
(72) Inventor: AN, Pengzhan, Sehnzhen, Guangdong 518101 (CN); WANG, Lingquan, Sehnzhen, Guangdong 518101 (CN); SUN, Congyang, Sehnzhen, Guangdong 518101 (CN); JIANG, Wennan, Sehnzhen, Guangdong 518101 (CN); ZHANG, Yuxuan, Sehnzhen, Guangdong 518101 (CN); LI, Weiguo, Sehnzhen, Guangdong 518101 (CN)
(74) Representative: Steffens, Adrian
(86) International application number: PCT/CN2024/115600
(87) International publication number: WO 2025/102902

(57) **Abstract**

Disclosed is a fragrance apparatus, belonging to the technical field of aromatherapy devices. The fragrance apparatus includes a fragrance component and a main component; the fragrance component includes a liquid storage bin, a liquid guide, a heater, and a first connector; the first connector is detachably connected to the main component; the liquid storage bin is provided with a closed liquid storage chamber for storing an essential oil, the liquid guide is in fluid communication with the liquid storage chamber, the liquid guide is used for transporting the essential oil in the liquid storage chamber, and the heater can heat the essential oil in the liquid guide to volatilize and diffuse the essential oil outward. In the fragrance apparatus, the first connector can be detachably connected to the main component, so that the fragrance component forms a replaceable and relatively independent module for sealing the essential oil therein; further, the essential oil is stored in the closed liquid storage chamber and is not in direct contact with air, and the essential oil is heated by the heater to volatilize and diffuse, so that the fragrance component can prevent waste caused by the volatilization of the essential oil and can also prevent the essential oil from being oxidized and deteriorated. (Fig. 3)

## Description

### CROSS REFERENCE TO RELATED APPLICATION

The present application claims priorities to Chinese Patent Applications No. 2023231221058 and No. 202311545636X filed on November 17, 2023, which are incorporated herein by reference in their entireties.

### TECHNICAL FIELD

The present application relates to the technical field of aromatherapy devices, in particular to a fragrance apparatus.

### BACKGROUND

Most existing aromatherapy machines have an open structure that allows essential oils to naturally volatilize and release fragrance. The essential oils in the aromatherapy machines are directly exposed to air. On the one hand, the volatilization states of the essential oils cannot be controlled, and the essential oils still volatilize when not in use to cause their waste. On the other hand, ingredients of the essential oils exposed to the air for a long term are easily oxidized and deteriorated, and the deteriorated essential oils will affect user experience.

### SUMMARY

The present application provides a fragrance apparatus, which can solve the technical problems of waste caused by volatilization of essential oils and easy oxidation and deterioration of the essential oils.

To solve the above technical problems, the present application provides a fragrance apparatus, including a fragrance component and a main component, the fragrance component including a liquid storage bin, a liquid guide, a heater, and a first connector, where the first connector can be detachably connected to the main component; the liquid storage bin is provided with a closed liquid storage chamber for storing an essential oil, the liquid guide is in fluid communication with the liquid storage chamber, the liquid guide is used for transporting the essential oil in the liquid storage chamber, and the heater can heat the essential oil in the liquid guide to volatilize and diffuse the essential oil outward; and under external force, the relative movement of the fragrance component and the main component triggers opening or closing of the fragrance apparatus.

In the fragrance apparatus provided by the present application, the first connector can be detachably connected to the main component, so that the fragrance component forms a replaceable and relatively independent module for sealing the essential oil inside the fragrance component; further, the essential oil is stored in the closed liquid storage chamber, the liquid guide transports the essential oil, and the heater heats the essential oil to volatilize and diffuse; on the one hand, the essential oil is sealed in the liquid storage chamber and is not in direct contact with air, which can prevent the essential oil from being oxidized and deteriorated; on the other hand, the essential oil needs to be heated by the heater to volatilize and diffuse, so the volatilization of the essential oil can be controlled by controlling the heating state of the heater, thereby preventing waste caused by the volatilization of the essential oil; therefore, the fragrance component can prevent waste caused by the volatilization of the essential oil, and can also prevent the essential oil from being oxidized and deteriorated.

### BRIEF DESCRIPTION OF THE DRAWINGS

To describe the technical solutions in the embodiments of the present application more clearly, the following briefly introduces the accompanying drawings required for use in the description of the embodiments. Apparently, the accompanying drawings in the following description show only some embodiments of the present application, and those of ordinary skill in the art can further derive other drawings from the accompanying drawings without any creative effort.
- FIG. 1: is a structure diagram of an embodiment of a fragrance component provided in the present application;
- FIG. 2: is an exploded structure diagram of an embodiment of the fragrance component provided in the present application;
- FIG. 3: is a cross-sectional structure diagram of an embodiment of the fragrance component provided by the present application from a perspective;
- FIG. 4: is a cross-sectional structure diagram of an embodiment of the fragrance component provided by the present application from another perspective;
- FIG. 5: is a structure diagram showing a heater is connected to a liquid guide in an embodiment of the fragrance component provided in the present application;
- FIG. 6: is an exploded structure diagram of an embodiment of a fragrance apparatus provided in the present application;
- FIG. 7: is a structure diagram of an embodiment of the fragrance apparatus provided in the present application in a closed state;
- FIG. 8: is a structure diagram of an embodiment of the fragrance apparatus provided in the present application in an open state;
- FIG. 9: is a cross-sectional structure diagram of a first embodiment of an aromatherapy apparatus provided in the present application;
- FIG. 10: is a cross-sectional structure diagram of a second embodiment of the aromatherapy apparatus provided in the present application;
- FIG. 11: is a structure diagram of an embodiment of a liquid guide member provided in the present application;
- FIG. 12: is a structure diagram of another embodiment of the liquid guide member provided in the present application;
- FIG. 13: is a structure diagram of still another embodiment of the liquid guide member provided in the present application;
- FIG. 14: is a structure diagram of yet another embodiment of the liquid guide member provided in the present application;
- FIG. 15: is an exploded structure diagram of a first embodiment of a liquid storage component provided in the present application;
- FIG. 16: is a cross-sectional structure diagram of the first embodiment of the liquid storage component provided in the present application;
- FIG. 17: is a partial cross-sectional structure diagram of a second embodiment of the liquid storage component provided in the present application;
- FIG. 18: is a partial cross-sectional structure diagram of a third embodiment of the liquid storage component provided in the present application;
- FIG. 19: is a partial cross-sectional structure diagram of a fourth embodiment of the liquid storage component provided in the present application;
- FIG. 20: is a partial cross-sectional structure diagram of a fifth embodiment of the liquid storage component provided in the present application;
- FIG. 21: is a partial cross-sectional structure diagram of a sixth embodiment of the liquid storage component provided in the present application;
- FIG. 22: is a structure diagram of an embodiment of a heating element provided in the present application;
- FIG. 23: is a structure diagram of an embodiment of a volatilization aid provided in the present application;
- FIG. 24: is an exploded structure diagram of a third embodiment of the aromatherapy apparatus provided in the present application;
- FIG. 25: is a cross-sectional structure diagram of the third embodiment of the aromatherapy apparatus provided in the present application; and
- FIG. 26: is a side view of the third embodiment of the aromatherapy apparatus provided in the present application from a perspective.

### DETAILED DESCRIPTION

The present application will be further described in detail below in conjunction with the accompanying drawings and embodiments. It should be particularly noted that the following embodiments are only used for illustrating the present application, but do not limit the scope of the present application. Similarly, the following embodiments are only some embodiments of the present application, not all embodiments. All other embodiments obtained by those of ordinary skill in the art without creative effort fall within the scope of protection of the present application.

In the description of the present application, "a plurality of" means at least two, such as two or three, unless otherwise specified. The terms "first", "second", and "third" used in the embodiments of the present application are for descriptive purposes only and should not be understood as indicating or implying relative importance or implying a quantity of indicated technical features. Therefore, the features limited to "first", "second", and "third" may explicitly or implicitly include at least one of these features. All directional indications (such as up, down, left, right, front, and back) in the embodiments of the present application are only used for explaining relative positional relationships, movement situations, and the like of components at a specific posture (as shown in the accompanying drawings), and if the specific posture changes, the directionality indication changes accordingly. The terms "include" and "have" and any variations thereof in the embodiments of the present application are intended to cover non-exclusive inclusions. For example, a process, method, system, product, or device that includes a series of steps or units is not limited to the listed steps or units, but optionally further includes unlisted steps or units, or optionally further includes other inherent steps or units of the process, method, product, or device.

The phrase "embodiment" referred to herein means that specific features, structures, or characteristics described in conjunction with the embodiment may be included in at least one embodiment of the present application. The phrase at various locations in the description does not necessarily refer to the same embodiment, or an independent or alternative embodiment exclusive of other embodiments. Those skilled in the art understand explicitly and implicitly that an embodiment described herein may be combined with another embodiment.

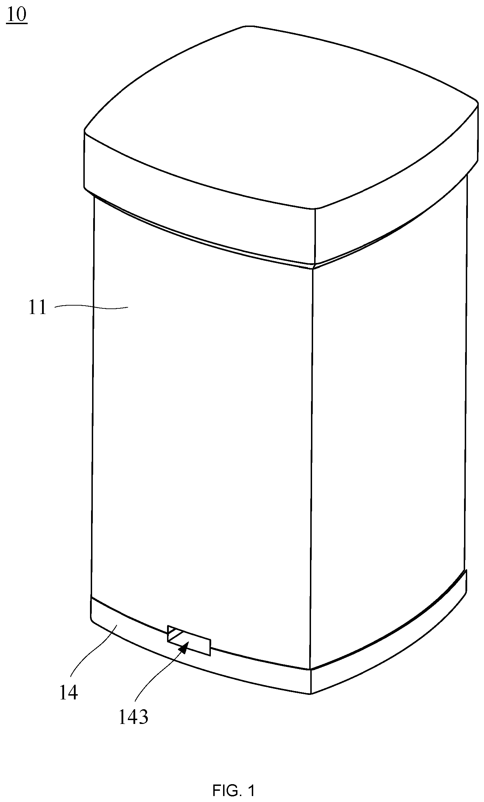

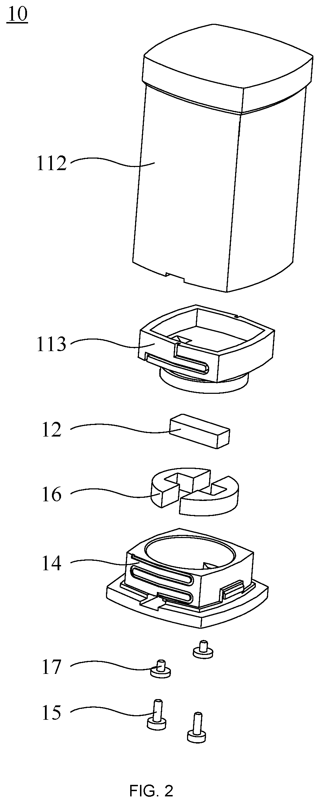

The present application provides a fragrance component. With reference to FIGs. 1-4, the fragrance component 10 may include a liquid storage bin 11, a liquid guide 12, a heater 13, and a first connector 17. The first connector 17 can be detachably connected to an external main component to facilitate the installation and removal of the fragrance component 10, so that the fragrance component 10 forms a replaceable and relatively independent module for sealing an essential oil inside the fragrance component 10. The liquid storage bin 11 is provided with a closed liquid storage chamber 111 for storing the essential oil. For example, the liquid storage bin 11 may be cylindrical, and its cross-section may be circular, elliptical, polygonal, or the like. Optionally, in the assembling process of the fragrance component 10, the essential oil may be first injected into the liquid storage chamber 111, and then the liquid storage chamber 111 is closed to seal the essential oil inside the fragrance component 10; or an oil injection hole for injecting the essential oil is formed on the liquid storage chamber 111, the essential oil can be injected into the liquid storage chamber 111 after assembly and before the product leaves the factory, or during use as required, and the oil injection hole is sealed to seal the essential oil inside the fragrance component 10.

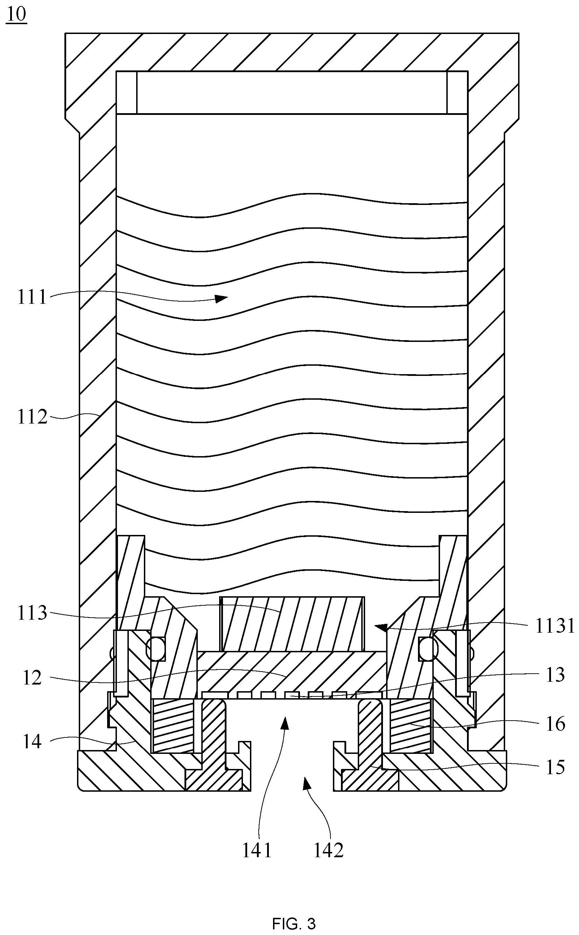

With continued reference to FIG. 3, the liquid guide 12 is in fluid communication with the liquid storage chamber 111, and the liquid guide 12 is used for transporting the essential oil in the liquid storage chamber 111. The liquid guide 12 is a porous medium. The liquid guide 12 can uniformly and quickly adsorb and transport the essential oil in the liquid storage chamber 111. By controlling the pore size of capillary micropores of the liquid guide 12, the oil guide rate of the liquid guide 12 can be adjusted. Meanwhile, the capillary micropores of the liquid guide 12 also have the function of locking oil to prevent the essential oil from leaking. The liquid guide 12 may be made of a material having good oil guide property and oil locking property, such as fiber cotton, ceramics, wood, or quartz glass, or may be formed from the above base material by secondary processing.

As shown in FIG. 3, the heater 13 can heat the essential oil in the liquid guide 12, so that the essential oil volatilizes and diffuses outward. The volatilization rate of the essential oil is positively correlated with its temperature, so the volatilization rate of the essential oil can be controlled by adjusting its temperature. Specifically, in an idle state of the fragrance component 10, the heater 13 can be controlled not to heat up. Due to the relatively low temperature of the essential oil in the liquid guide 12, the volatilization rate of the essential oil is slow, and the volatilization surface of the liquid guide 12 is usually small. Therefore, the essential oil in the liquid guide 12 almost does not volatilize and is sealed in the liquid storage chamber 111, which can prevent waste caused by the volatilization of the essential oil. In a working state of the fragrance component 10, the heater 13 heats the essential oil in the liquid guide 12, the temperature of the essential oil rises to increase its volatilization rate, and the essential oil can volatilize normally and diffuse outward.

In the fragrance component 10 provided by the present application, the first connector 17 can be detachably connected to the external main component, so that the fragrance component 10 forms a replaceable and relatively independent module for sealing the essential oil inside the fragrance component 10; further, the essential oil is stored in the closed liquid storage chamber 111, the liquid guide 12 transports the essential oil, and the heater 13 heats the essential oil to volatilize and diffuse; on the one hand, the essential oil is sealed in the liquid storage chamber 111 and is not in direct contact with air, which can prevent the essential oil from being oxidized and deteriorated; on the other hand, the essential oil needs to be heated by the heater 13 to volatilize and diffuse, so the volatilization of the essential oil can be controlled by controlling the heating state of the heater 13, thereby preventing waste caused by the volatilization of the essential oil; therefore, the fragrance component 10 can prevent waste caused by the volatilization of the essential oil, and can also prevent the essential oil from being oxidized and deteriorated.

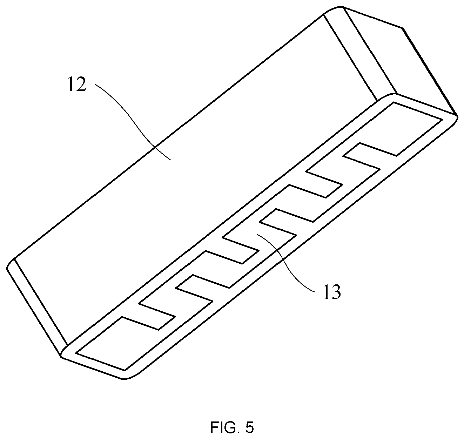

Optionally, the heater 13 is spaced apart from the liquid guide 12, and the heater 13 heats the essential oil in the liquid guide 12 through thermal radiation; or the heater 13 is connected to the liquid guide 12, as shown in FIG. 5, which facilitates the assembly of the heater 13 and the liquid guide 12 as a whole, reduces the quantity of materials during assembly, and can improve production efficiency. Optionally, the heater 13 implements heating by resistance heating, and the heater 13 can be attached to the liquid guide 12 by a process of screen printing, PVD coating, electroplating, chemical plating, or the like. In different usage environments of fragrance component 10, different types of essential oils are usually required, and the different types of essential oils have different optimal fragrance emitting temperatures. By configuring the heater 13 to heat the essential oils, the temperatures of the essential oils can be adjusted to meet the optimal fragrance emitting temperatures of the different types of essential oils.

In one embodiment, as shown in FIG. 3, the liquid storage bin 11 includes a bin shell 112 and a bracket 113. The bin shell 112 is of a cylindrical structure with an open end, the bracket 113 is connected to the open end of the bin shell 112, and the bracket 113 is accommodated inside the bin shell 112. The bracket 113 is provided with an oil supply hole 1131. The liquid guide 12 is mounted on the bracket 113, the liquid guide 12 is at least partially blocked at the oil supply hole 1131, and the liquid storage chamber 111 is enclosed by the bin shell 112, the bracket 113, and the liquid guide 12. The liquid guide 12 is in fluid communication with the liquid storage chamber 111 through the oil supply hole 1131, so that the essential oil in the liquid storage chamber 111 can be transferred to the liquid guide 12. The liquid storage chamber 111 is enclosed by the bin shell 112, the bracket 113, and the liquid guide 12, so that the liquid storage chamber 111 is relatively closed, which can prevent waste caused by the volatilization of the essential oil and also prevent the essential oil from being oxidized and deteriorated compared to an open structure.

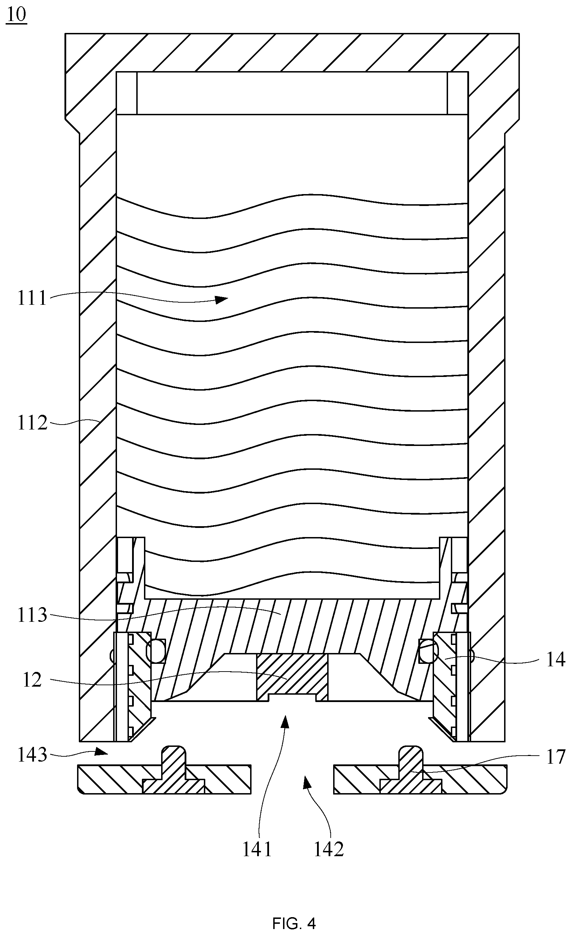

With reference to FIG. 3 and FIG. 4, in one embodiment, the fragrance component 10 further includes a base 14 and an electrode 15. The base 14 is connected to the open end of the bin shell 112, the bracket 113 is partially accommodated inside the base 14, and the liquid guide 12 is mounted inside a volatilization chamber 141 enclosed by the base 14 and the bracket 113. A bottom of the base 14 is provided with an air inlet 142, a side wall of the base 14 is provided with a volatilization hole 143, both the air inlet 142 and the volatilization hole 143 are in communication with the volatilization chamber 141, and the essential oil volatilized from the liquid guide 12 can be released and diffused to the outside of the fragrance component 10 via the volatilization hole 143. By configuring the air inlet 142, external airflow can be introduced into the volatilization chamber 141 through the air inlet 142, thereby accelerating the diffusion of the essential oil in the volatilization chamber 141. The electrode 15 is inserted into the base 14, and the electrode 15 is electrically connected to the heater 13, so that the heater 13 can be connected to an external power source.

A sealing member may be provided at the connection between the bracket 113 and the bin shell 112 and between the bracket and the base 14 to improve the sealing property of the fragrance component 10. The sealing member may be made of silicone or rubber. The silicone or rubber has good compressibility. When the sealing members are assembled between the bracket 113 and the bin shell 112 and between the bracket 113 and the base 14 by interference fit, the sealing members undergo compression deformation to seal assembly gaps, thereby enhancing the sealing property of the fragrance component 10.

During the transportation of the fragrance component 10, if the liquid storage chamber 111 is injected with an essential oil, when the external environment such as air pressure, temperature, or humidity changes, the essential oil saturation adsorption rate of the liquid guide 12 will change, and some essential oil may leak. In one embodiment, as shown in FIG. 3, the fragrance component 10 includes a liquid absorber 16, the liquid absorber 16 is mounted on the base 14 and accommodated in the volatilization chamber 141, the liquid absorber 16 is spaced apart from the liquid guide 12, and the liquid absorber 16 can adsorb the essential oil leaking from the liquid guide 12. When the essential oil leaks from the liquid guide 12, the essential oil flows towards the base 14 under the action of gravity. The liquid absorber 16 is mounted on the base 14, and the liquid absorber 16 is arranged on a flow path of the essential oil, so that the essential oil can be adsorbed and accumulated in the liquid absorber 16, thereby preventing the essential oil from flowing to the outside of the fragrance component 10. The liquid absorber 16 may be made of a fiber material with strong adsorption capacity, such as fiber cotton. The liquid absorber 16 can adsorb the essential oil leaking from the liquid guide 12, thereby preventing leakage of the essential oil.

The fragrance component 10 provided in the present application forms a replaceable and relatively independent module, which facilitates sealing of the essential oil inside the fragrance component 10 to prevent waste caused by the volatilization of the essential oil and also prevent the essential oil from being oxidized and deteriorated. In use, the fragrance component 10 can be connected and matched with the main component. Optionally, the connection method between the first connector 17 and the external main component may be one of adhesion, clamping, or threaded connection. Correspondingly, the first connector 17 may be a double-sided adhesive tape, clamping joint, or bolt. In one embodiment, the first connector 17 is arranged at one end of the fragrance component 10, the first connector 17 has magnetism, and the first connector 17 is used for magnetic connection with the external main component. The first connector 17 has magnetism, so that the first connector 17 can be magnetically connected to the external main component. Relatively speaking, the magnetic connection has the advantages of easy disassembly and assembly, secure connection, etc. For example, the first connector 17 is inserted into the base 14, which can reduce the occupation of the internal space of the fragrance component 10 by the first connector 17 and facilitate the magnetic connection between the fragrance component 10 and the external main component.

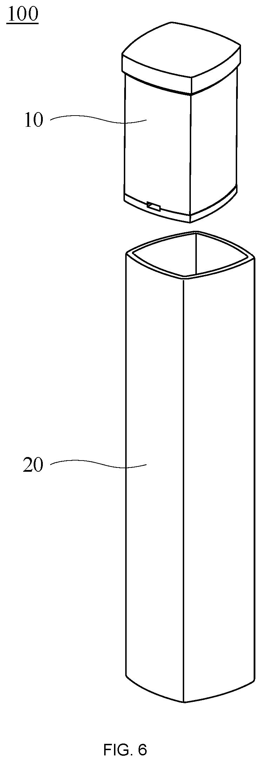

The present application provides a fragrance apparatus. With reference to FIGs. 6-8, the fragrance apparatus 100 may include the fragrance component 10 as described above and a main component 20. The fragrance component 10 is detachably mounted on the main component 20. The main component 20 is provided with a second connector 26, and the second connector 26 is detachably connected to the first connector 17, so that the fragrance component 10 forms a replaceable and relatively independent module. The main component 20 can control the fragrance component 10 to heat the essential oil, so that the essential oil volatilizes and diffuses.

The fragrance component 10 is detachably connected to the main component 20. On the one hand, a user can change the fragrance component 10 with new ones containing different types of essential oils to adapt to different usage environments, thereby expanding the usage scenario of the fragrance apparatus 100. On the other hand, when the essential oil in the fragrance component 10 is used up, the fragrance component 10 can be replaced for continued use, without replacing the main component 20, thereby reducing usage costs. In addition, the fragrance component 10 forms a replaceable and relatively independent module, which facilitates sealing of the essential oil inside the fragrance component 10 to prevent waste caused by the volatilization of the essential oil and also prevent the essential oil from being oxidized and deteriorated.

Optionally, the connection method between the second connector 26 and the first connector 17 may be one of adhesion, clamping, threaded connection, or magnetic connection. Correspondingly, the second connector 26 may be an adhesive plate, clamping seat, nut, or magnet corresponding to the first connector 17.

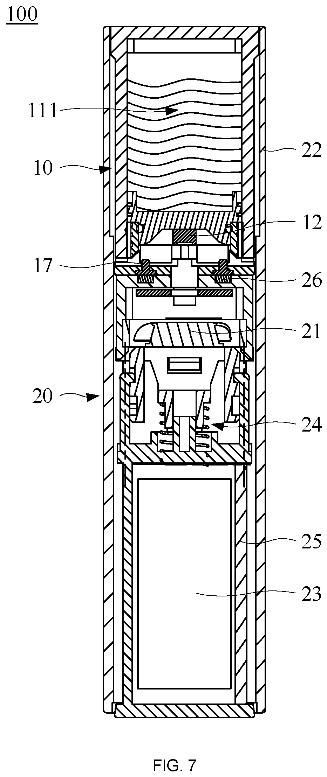

With reference to FIG. 7, in one embodiment, the main component 20 includes a diffusion aid 21, the diffusion aid 21 is mounted at an end, close to the liquid guide 12, of the main component 20, the diffusion aid 21 can produce an airflow, and the airflow passes through the liquid guide 12 and drives the essential oil volatilized from the liquid guide 12 to diffuse outward. For example, the diffusion aid 21 is spaced apart from the liquid guide 12 in a longitudinal direction of the fragrance apparatus 100. The airflow produced by the diffusion aid 21 flows in the direction of the liquid guide 12, and the airflow can enter the volatilization chamber 141 from the air inlet 142 and flow through the liquid guide 12, thereby driving the essential oil volatilized from the liquid guide 12 to diffuse and improve the diffusion rate.

Optionally, the diffusion aid 21 may be a fan or air pump. When the fan or air pump is working, strong airflow can be produced, thereby accelerating the diffusion of the essential oil.

In one embodiment, as shown in FIG. 7, the main component 20 further includes an outer shell 22, a battery 23, a start control module 24, and a circuit board 25. The fragrance component 10 is mounted at one end of the outer shell 22; and the battery 23, the start control module 24, and the circuit board 25 are mounted at an opposite end, away from the fragrance component 10, of the outer shell 22. The battery 23 can provide working power for the heater 13, and the circuit board 25 can control the heating mode of the heater 13. Generally, different types of essential oils have different optimal fragrance emitting temperatures. The circuit board 25 can adjust the heating temperature of the heater 13 according to the characteristics of an essential oil, so as to adjust the temperature of the essential oil to the optimal fragrance emitting temperature, which can meet the optimal fragrance emitting temperatures of the different types of essential oils.

Under external force, the start control module 24 can control the connection or disconnection of the fragrance component 10 and the battery 23 to turn on or off the fragrance apparatus 100. For example, the external force may be pressing, such as pressing the fragrance component 10, and the fragrance component 10 moves relative to the main component 20 to trigger the connection or disconnection of the fragrance component 10 and the battery 23.

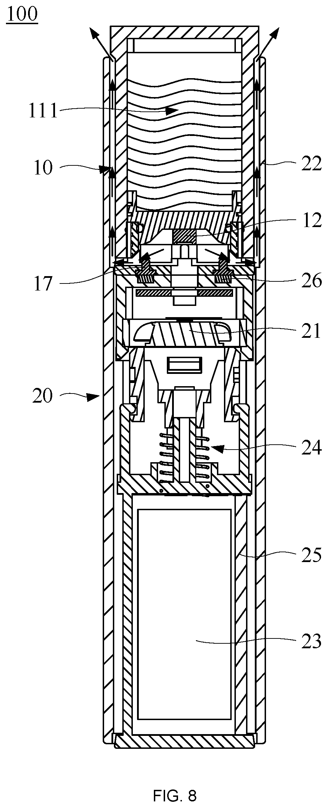

With reference to FIG. 8, in the working state, the essential oil volatilized from the fragrance component 10 can be released and diffused to the outside of the fragrance apparatus 100 via the gap between the fragrance component 10 and the outer shell 22. For example, by pressing the fragrance component 10, the fragrance component 10 moves relative to the main component 20, the fragrance component 10 is partially exposed to the outer shell 22, the gap between the fragrance component 10 and the outer shell 22 forms a fragrance outlet, the fragrance component 10 is connected to the battery 23, the fragrance apparatus 100 is turned on, and the essential oil volatilized from the fragrance component 10 can be released and diffused to the outside of the fragrance apparatus 100 via the gap between the fragrance component 10 and the outer shell 22, where the diffusion path of the essential oil is shown by arrows in the figure. By pressing the fragrance component 10 again, the fragrance component 10 moves relative to the main component 20, the fragrance component 10 is accommodated in the outer shell 22 by the pressing, the fragrance component 10 is attached to the outer shell 22, the fragrance outlet is closed, the fragrance component 10 is disconnected from the battery 23, and the fragrance apparatus 100 is turned off, as shown in FIG. 7. The operation is repeated, so that when triggered by an external action, the start control module 24 can control the fragrance apparatus 100 to switch between opening and closing.

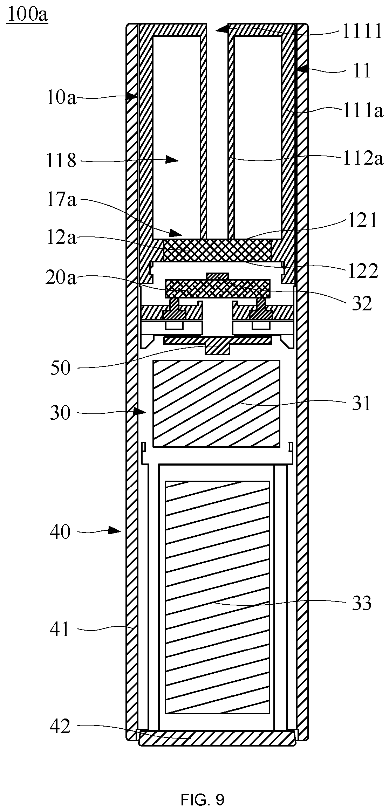

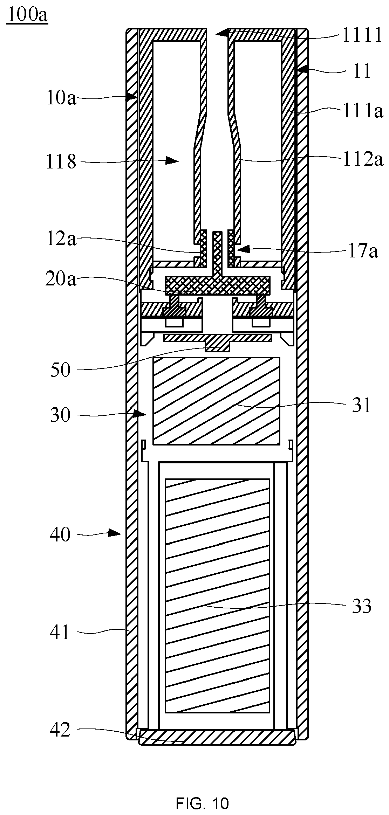

The present application provides an aromatherapy apparatus. With reference to FIG. 9 and FIG. 10, the aromatherapy apparatus 100a may include a liquid storage component 10a and a heating element 20a. The aromatherapy apparatus 100a may be equivalent to the aforementioned fragrance apparatus 100, the liquid storage component 10a may be equivalent to the aforementioned fragrance component 10, and the heating element 20a may be equivalent to the aforementioned heater 13. The liquid storage component 10a includes a liquid storage bin 11 and a liquid guide member 12a, the liquid storage bin 11 is provided with a sealed liquid storage chamber 118, and the liquid storage chamber 118 is used for storing an essential oil. The liquid guide member 12a may be equivalent to the aforementioned liquid guide 12. For example, the liquid storage bin 11 may be cylindrical, and its cross-section may be circular, elliptical, polygonal, or the like. The liquid guide member 12a is in fluid communication with the liquid storage chamber 118, and the liquid guide member 12a can transport the essential oil in the liquid storage chamber 118. With continued reference to FIG. 9 and FIG. 10, the heating element 20a is spaced apart from the liquid guide member 12a, and the heating element 20a can heat the essential oil in the liquid guide member 12a to volatilize and diffuse the essential oil to an external environment. That the heating element 20a is spaced apart from the liquid guide member 12a indicates that a minimum spacing between the heating element 20a and the liquid guide member 12a is greater than zero, and the two are not in contact with each other to prevent local heating of the essential oil in the liquid guide member 12a.

In the aromatherapy apparatus 100a provided by the present application, the liquid storage bin 11 is provided with the sealed liquid storage chamber 118 for storing an essential oil, the liquid guide member 12a is in fluid communication with the liquid storage chamber 118, the heating element 20a is spaced apart from the liquid guide member 12a, and the essential oil in the liquid guide member 12a is heated by the heating element 20a to volatilize and diffuse to the external environment. Through the above settings, the essential oil is sealed in the liquid storage chamber 118 and is not in contact with external air before being heated to volatilize and diffuse, which can prevent the essential oil from being deteriorated due to long-term contact with the air.

Experiments show that the volatilization rate of the essential oil is positively correlated with its temperature. Therefore, the temperature of the essential oil can be changed by the heating element 20a to adjust the volatilization rate of the essential oil, so that the aromatherapy apparatus 100a is suitable for different usage environments requiring the volatilization rate of essential oils and can meet the requirements of various usage scenarios. In related technologies, the heating element is placed in the essential oil and is in direct contact with the heating element, the essential oil in contact with the heating element may be locally heated and ingredients therein may be damaged to some extent, so that the essential oil produces an unpleasant odor and the fragrance of the essential oil cannot be truly restored. In the aromatherapy apparatus 100a provided by the present application, the heating element 20a is spaced apart from the liquid guide member 12a, and the heating element 20a heats the essential oil in the liquid guide member 12a by non-contact radiation heating, so the essential oil in the liquid guide member 12a is heated more uniformly, the ingredients in the essential oil will not be damaged due to local heating, and the fragrance of the essential oil can be truly restored.

In one embodiment, as shown in FIG. 9 and FIG. 10, the liquid storage bin 11 includes a bin shell 111a and a volatilization tube 112a, and the volatilization tube 112a is mounted inside the bin shell 111a. One end of the bin shell 111a is provided with a first volatilization hole 1111, the volatilization tube 112a is in communication with the first volatilization hole 1111, and the essential oil volatilized from the liquid guide member 12a can diffuse to the outside of the aromatherapy apparatus 100a via the volatilization tube 112a and the first volatilization hole 1111. The volatilization tube 112a may be integrally formed with the bin shell 111a, or the two may be separately machined and then assembled and connected, which is not limited here. By configuring the volatilization tube 112a, the volatilization tube 112a can guide the essential oil to diffuse to the outside of the aromatherapy apparatus 100a, making the diffusion of the essential oil smoother.

With reference to FIGs. 9-12, the liquid guide member 12a has a liquid guide surface 121 and a volatilization surface 122, the liquid guide surface 121 is in fluid communication with the liquid storage chamber 118, the volatilization surface 122 faces a heating part of the heating element 20a at a preset distance, and the essential oil entering the liquid guide member 12a from the liquid guide surface 121 is heated on the volatilization surface 122 and volatilized to the external environment. Optionally, the liquid guide surface 121 is one of the surfaces of the liquid guide member 12a, and the volatilization surface 122 may be another surface opposite to the liquid guide surface 121, that is, the liquid guide surface 121 is opposite to the volatilization surface 122, making the shape of the liquid guide member 12a regular to facilitate machining.

In one embodiment, as shown in FIG. 9 and FIG. 10, the liquid storage bin 11 is provided with an oil outlet through hole 17a, and the liquid guide member 12a is connected to the liquid storage bin 11, where the liquid guide surface 121 faces the oil outlet through hole 17a to transfer the essential oil from the oil outlet through hole 17a to the volatilization surface 122. The essential oil is transferred to the liquid guide member 12a through the oil outlet through hole 17a, so that the cross-sectional area of the oil outlet through hole 17a can be controlled to adjust the supply rate of the essential oil to match the volatilization rate on the volatilization surface 122.

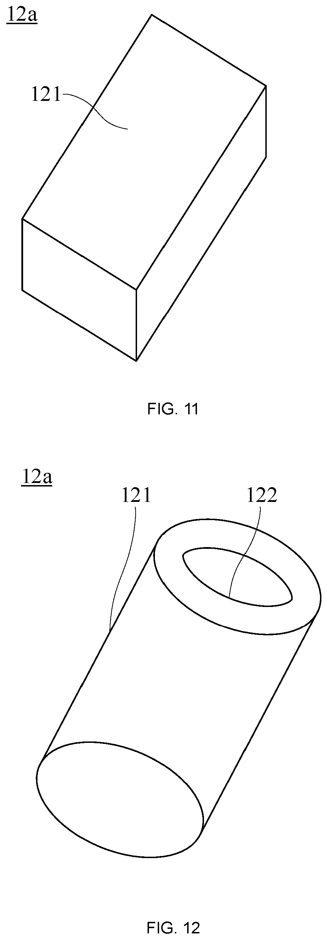

The liquid guide member 12a may be configured in many ways. Optionally, in one embodiment, as shown in FIG. 9 and FIG. 11, the liquid guide member 12a is block-shaped. For example, the liquid guide member 12a may be cuboid. The liquid guide member 12a is mounted at an end, away from the first volatilization hole 1111, of the liquid storage bin 11. For example, the oil outlet through hole 17a may be formed at a bottom of the liquid storage bin 11, and the liquid guide member 12a is at least partially blocked at the oil outlet through hole 17a to form the sealed liquid storage chamber 118. The liquid guide member 12a is at least partially blocked at the oil outlet through hole 17a, so that at least a portion of the liquid guide member 12a can be exposed to the liquid storage chamber 118 via the oil outlet through hole 17a, to transport the essential oil in the liquid storage chamber 118 to the heating element 20a. The width of the liquid guide member 12a may be smaller than the diameter of the volatilization tube 112a. For example, the width direction may be perpendicular to the cross-section in FIG. 9, so that there are gaps between two sides of the liquid guide member 12a in the width direction and the volatilization tube 112a, and the essential oil volatilized from the liquid guide member 12a can enter the volatilization tube 112a via the gaps. The heating element 20a can perform radiation heating on the volatilization surface 122 to heat the essential oil in the liquid guide member 12a, so that the volatilization rate of the essential oil can be adjusted by changing the temperature of the essential oil.

With reference to FIG. 10 and FIG. 12, in one embodiment, the liquid guide member 12a is in the shape of a hollow column, an outer wall of the liquid guide member 12a is the liquid guide surface 121, and an inner wall of the liquid guide member 12a is the volatilization surface 122. The volatilization tube 112a is hollow, the liquid guide member 12a is accommodated inside the volatilization tube 112a, and the oil outlet through hole 17a is formed on the volatilization tube 112a, so that the liquid guide surface 121 is in fluid communication with the liquid storage chamber 118. The heating portion of the heating element 20a is accommodated inside the volatilization tube 112a, and the heating element 20a is spaced apart from the inner wall of the liquid guide member 12a. The heating element 20a can perform radiation heating on the inner wall of the liquid guide member 12a to heat the essential oil in the liquid guide member 12a, so that the volatilization rate of the essential oil can be adjusted by changing the temperature of the essential oil.

The liquid guide member 12a is made of a porous material, a capillary microporous structure is formed between the liquid guide surface 121 and the volatilization surface 122, and the essential oil is transported by the capillary microporous structure. By controlling the pore size of capillary micropores of the liquid guide member 12a, the oil guide rate of the liquid guide member 12a can be adjusted. Meanwhile, the capillary micropores of the liquid guide member 12a also have the function of locking oil to prevent the essential oil from leaking. The liquid guide member 12a may be made of a material having good oil guide property and oil locking property, such as fiber cotton, ceramics, wood, or quartz glass, or may be formed from the above base material by secondary processing.

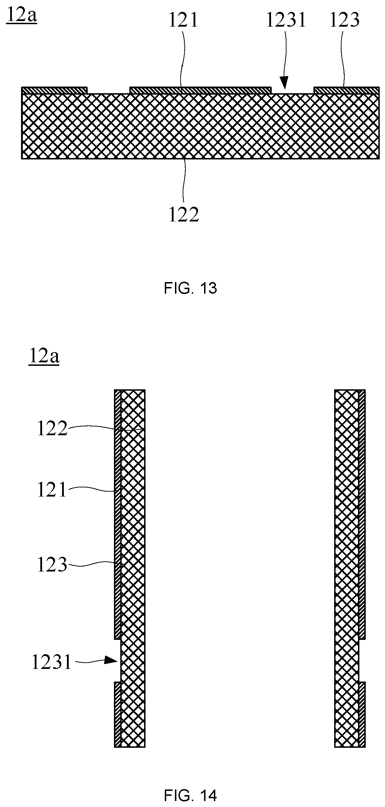

In one embodiment, as shown in FIG. 13 and FIG. 14, the liquid guide member 12a includes a sealing layer 123, the sealing layer 123 is attached to a peripheral surface of the liquid guide surface 121, the sealing layer 123 is provided with a liquid guide hole 1231, and the liquid guide hole 1231 is in fluid communication with the liquid storage chamber 118. The sealing layer 123 can seal a metal coating, a metal shell, or other sealable structure on the liquid guide surface 121. The sealing layer 123 is attached to the periphery of the liquid guide surface 121, and the sealing layer 123 can seal the essential oil inside the liquid guide member 12a, thereby preventing leakage of the liquid guide member 12a. In addition, only the liquid guide hole 1231 is in fluid communication with the liquid storage chamber 118, and the sealing layer 123 attached to other regions of the liquid guide surface 121 except the liquid guide hole 1231 is not in fluid communication with the liquid storage chamber 118, so the cross-sectional area of the liquid guide hole 1231 can be controlled to adjust the supply rate of the essential oil to match the volatilization rate of the volatilization surface 122.

Optionally, when the liquid guide member 12a is block-shaped, the sealing layer 123 can be attached to the surface where the liquid guide surface 121 is located, as shown in FIG. 13, or the sealing layer 123 can be attached to a plurality of other surfaces except the volatilization surface 122; and when the liquid guide member 12a is in the shape of a hollow column, the sealing layer 123 can be wrapped on the peripheral surface of the liquid guide surface 121, as shown in FIG. 14.

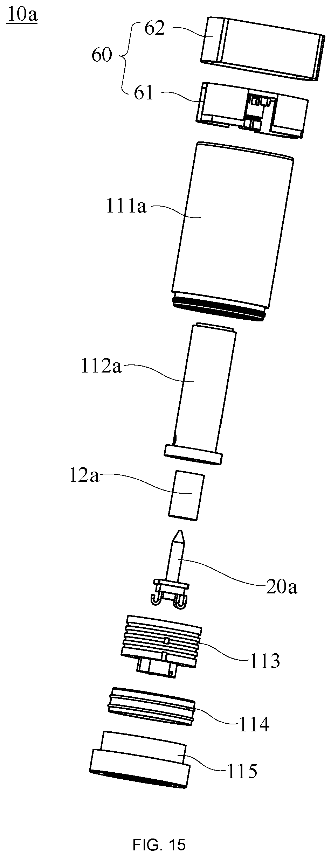

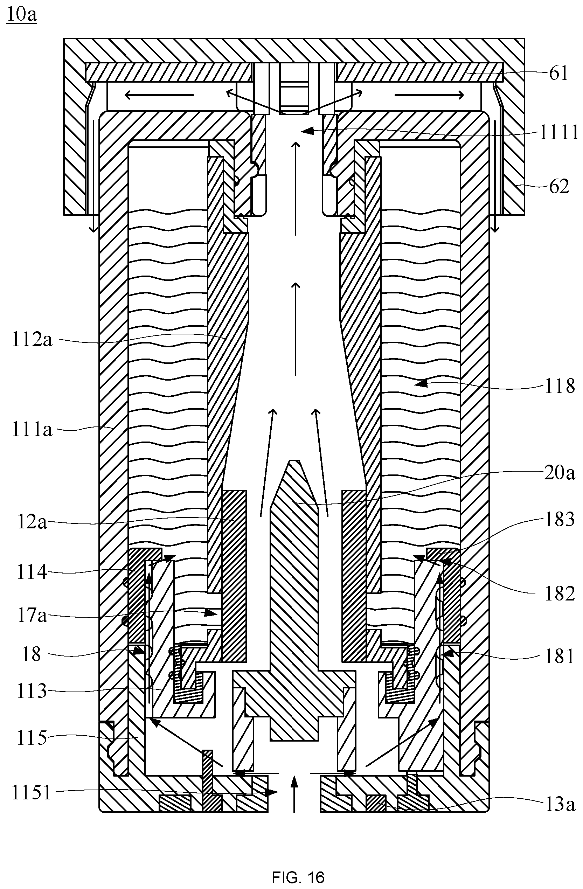

The essential oil flows towards the liquid guide member 12a under the action of gravity, thereby supplying the essential oil to the liquid guide member 12a in a timely manner and ensuring sustainable volatilization. The supply rate of the essential oil is related to the remaining quantity of essential oil in the liquid storage chamber 118. In the early stage of use, the quantity of essential oil in the liquid storage chamber 118 is large, the supply rate of the essential oil is also large, and leakage easily occurs. After a period of use, as the consumption of the essential oil decreases, the supply rate of the essential oil decreases, insufficient supply of the essential oil easily occurs, and the volatilization rate changes accordingly. To adjust the supply rate of the essential oil, in one embodiment, as shown in FIG. 15 and FIG. 16, the liquid storage component 10a is provided with a pressure balancing structure 18, the pressure balancing structure 18 balances the pressure inside the liquid storage chamber 118 to maintain consistency with the external environment, and in at least one conventional working posture, the position of the oil outlet through hole 17a is lower than an air inlet 182 of the pressure balancing structure 18. The conventional working posture indicates that the essential oil in the liquid guide member 12a is volatilized and diffused to the external environment under the heating of the heating element 20a. For example, the aromatherapy apparatus 100a may be in a vertical state with the first volatilization hole 1111 facing upwards. External air can enter the liquid storage chamber 118 through the air inlet 182 of the pressure balancing structure 18, and the essential oil in the liquid storage chamber 118 will not leak from the pressure balancing structure 18, thereby adjusting the gas pressure inside the liquid storage chamber 118 to maintain a stable supply rate of the essential oil.

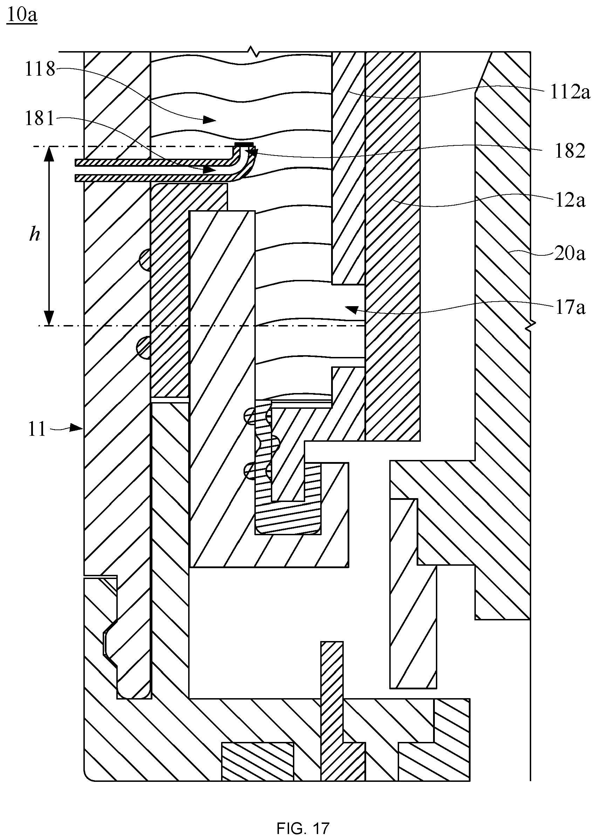

Specifically, the air inlet 182 faces the interior of the liquid storage chamber 118, and external air can enter the liquid storage chamber 118 through the air inlet 182. During the use of the aromatherapy apparatus 100a, the essential oil stored in the liquid storage chamber 118 gradually decreases due to volatilization and consumption, and the oil pressure produced by the essential oil at the air inlet 182 will decrease. At this time, the pressure of the external air is greater than the oil pressure produced by the essential oil, and the external air can enter the liquid storage chamber 118 through the air inlet 182 to increase the gas pressure inside the liquid storage chamber 118. Under the joint action of the gas pressure inside the liquid storage chamber 118 and the hydraulic pressure produced by the essential oil, the pressure at the air inlet 182 is equal to that of the external air, and the essential oil in the liquid storage chamber 118 is always in a dynamic state of force balance. Because the air inlet 182 can adjust the gas pressure inside the liquid storage chamber 118, the pressure at the air inlet 182 is always equal to that of the external air. Therefore, the pressure at the oil outlet through hole 17a depends only on the height difference h between the air inlet 182 and the oil outlet through hole 17a, as shown in FIGs. 17-20. The constant height difference h can keep the supply oil pressure of the essential oil inside the liquid storage chamber 118 stable, thereby ensuring a constant supply rate of the essential oil.

In one embodiment, as shown in FIG. 16, the pressure balancing structure 18 includes an air inlet passage 181, the air inlet passage 181 is connected to the liquid storage chamber 118 and the external environment, the air inlet 182 is arranged at the connection between the air inlet passage 181 and the liquid storage chamber 118, and a control valve 183 is provided at the air inlet 182 for ventilation and prevention of essential oil leakage. The control valve 183 can be blocked at the air inlet 182 to seal the liquid storage chamber 118. The control valve 183 allows the external air to enter the liquid storage chamber 118 through the air inlet 182, and the essential oil in the liquid storage chamber 118 will not leak from the air inlet 182, thereby adjusting the gas pressure inside the liquid storage chamber 118. For example, the control valve 183 can be movably blocked at the air inlet 182, and the control valve 183 is configured to open unidirectionally towards the liquid storage chamber 118; when the pressure at the air inlet 182 is lower than that of the external air, under the action of external air pressure, the control valve 183 can open towards a side where the liquid storage chamber 118 is located, so that the external air enters the liquid storage chamber 118 through the air inlet 182 until the essential oil in the liquid storage chamber 118 is in a state of force balance; and the control valve 183 closes the air inlet 182 to seal the essential oil inside the liquid storage chamber 118 to prevent leakage. Alternatively, the control valve 183 is fixedly attached to the air inlet 182, the control valve 183 is made of an oil resistant and air-permeable material, the control valve 183 allows external air to enter the liquid storage chamber 118 to balance the oil pressure, but the essential oil cannot pass through the control valve 183, so that the control valve 183 can ventilate and prevent leakage of the essential oil.

The air inlet passage 181 may be configured in many ways. Optionally, in one embodiment, as shown in FIG. 17, a pipe is inserted at a bottom of the liquid storage bin 11, the pipe forms the air inlet passage 181, the pipe is partially located inside the liquid storage chamber 118, an end of the pipe inside the liquid storage chamber 118 is bent upward, the air inlet 182 is arranged at the end of the pipe, and external air can enter the liquid storage chamber 118 through the air inlet 182 to increase the gas pressure inside the liquid storage chamber 118.

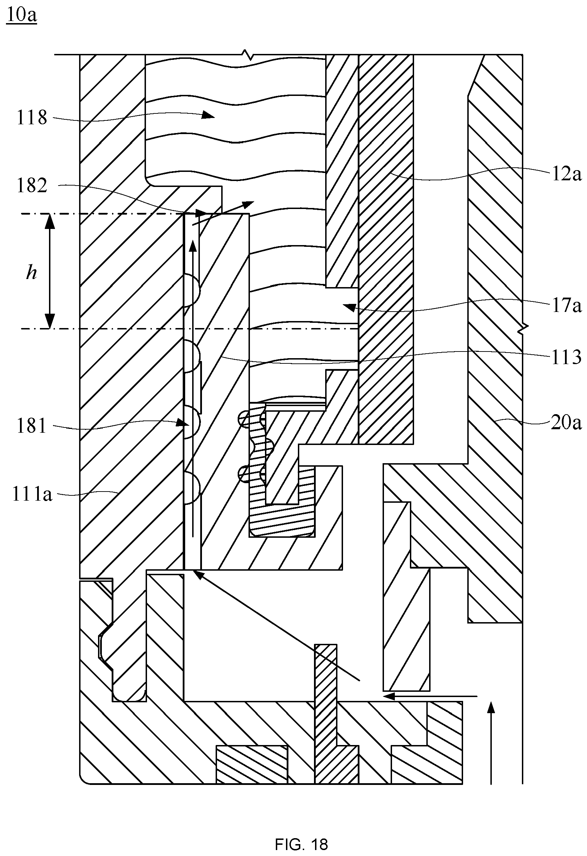

With reference to FIG. 18, in one embodiment, the liquid storage bin 11 includes a bin shell 111a and a bracket 113. The bracket 113 is connected to one end of the bin shell 111a, the bracket 113 is at least partially accommodated inside the bin shell 111a, the air inlet passage 181 is enclosed between the bracket 113 and the bin shell 111a, and the air inlet 182 is arranged at the connection between the air inlet passage 181 and the liquid storage chamber 118. For example, the air inlet 182 is located between an end of the bracket 113 and an inner wall of the bin shell 111a. The air inlet passage 181 is enclosed by the bracket 113 and the bin shell 111a, so compared to the air inlet passage 181 formed by the pipe, on the one hand, the quantity of materials is reduced and assembly can be facilitated; and on the other hand, a hole through which the pipe passes is reduced on the liquid storage bin 11, so the sealing property of the liquid storage bin 11 is enhanced, which is beneficial to preventing the essential oil from leaking.

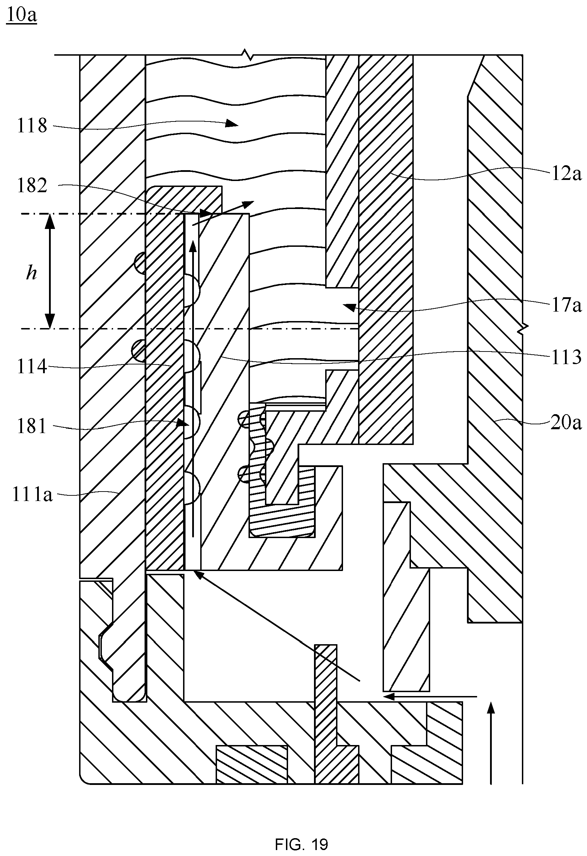

With reference to FIG. 19, in one embodiment, the liquid storage bin 11 includes a bin shell 111a, a bracket 113, and a sealing member 114. The bracket 113 is connected to one end of the bin shell 111a, the bracket 113 is at least partially accommodated inside the bin shell 111a, the sealing member 114 is at least partially attached between the bin shell 111a and the bracket 113, the air inlet passage 181 is enclosed between the bracket 113 and the sealing member 114, and the air inlet 182 is arranged at the connection between the air inlet passage 181 and the liquid storage chamber 118. For example, the air inlet 182 is located between an end of the bracket 113 and an inner wall of the sealing member 114. The sealing member 114 is made of a compressible elastic material, such as silicone or plastic. When the sealing member 114 is attached between the bin shell 111a and the bracket 113 by interference fit, the sealing member 114 undergoes elastic compression deformation, which can enhance the sealing property of the liquid storage bin 11 and prevent leakage. The air inlet passage 181 is enclosed by the bracket 113 and the sealing member 114, where the sealing member 114 can enhance the sealing property of the liquid storage bin 11, which is conducive to preventing the essential oil from leaking.

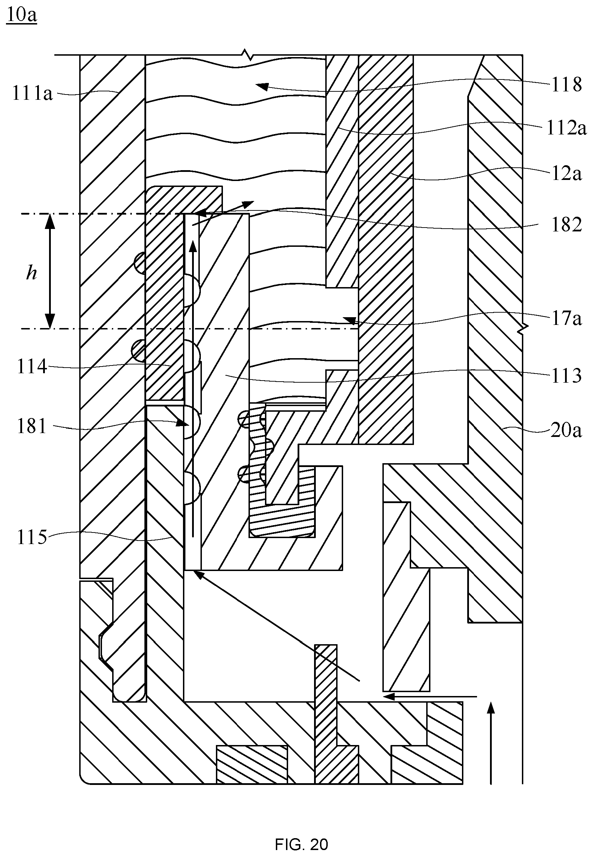

With reference to FIG. 16 and FIG. 20, in one embodiment, the liquid storage bin 11 includes a bin shell 111a, a bracket 113, a sealing member 114, and a base 115. The base 115 is connected to one end of the bin shell 111a, the bracket 113 is at least partially accommodated inside the base 115, and the sealing member 114 is at least partially attached between the bin shell 111a and the bracket 113 to ensure the sealing property of the liquid storage bin 11 and prevent leakage. The air inlet passage 181 is enclosed between the bracket 113 and the base 115 as well as the sealing member 114, the air inlet passage 181 connects the liquid storage chamber 118 with the external environment, and the air inlet 182 is arranged at the connection between the air inlet passage 181 and the liquid storage chamber 118. For example, the air inlet 182 is located between an end of the bracket 113 and an inner wall of the sealing member 114. The base 115 is provided with an air inlet 1151, and the air inlet 1151 is connected to the air inlet passage 181 and the external air. The air inlet passage 181 is enclosed between the bracket 113 and the base 115 as well as the sealing member 114, and the base 115 encapsulates the bracket 113, the sealing member 114, and other components within the bin shell 111a, so that the liquid storage bin 11 forms a relatively independent module, and the liquid storage bin 11 can be regarded as a whole for assembly.

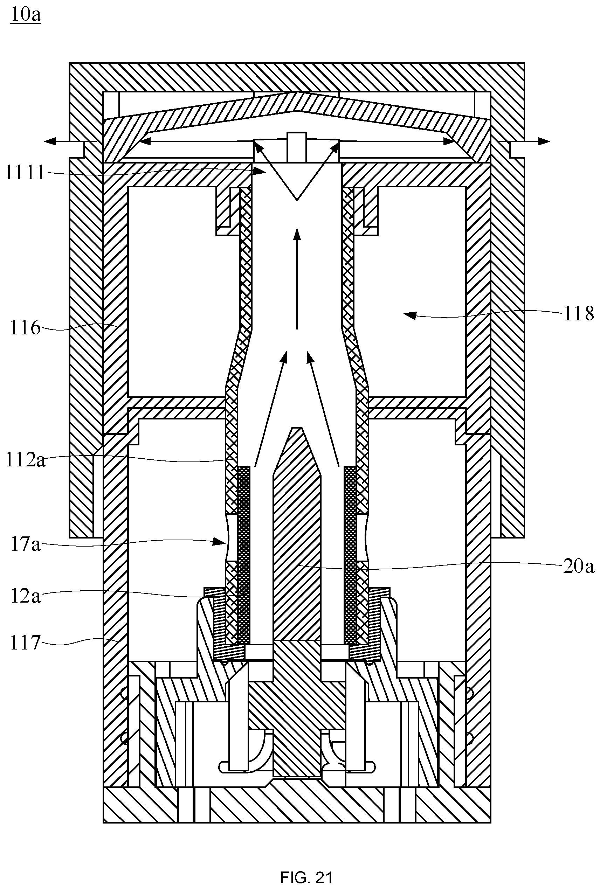

In one embodiment, as shown in FIG. 21, the liquid storage bin 11 includes a first liquid storage bin 116 and a second liquid storage bin 117, the liquid storage chamber 118 is arranged at the first liquid storage bin 116, the liquid guide member 12a is accommodated in the second liquid storage bin 117, and the second liquid storage bin 117 can be selectively connected to the first liquid storage bin 116, so that the essential oil enters the second liquid storage bin 117 from the first liquid storage bin 116, or the second liquid storage bin 117 seals the essential oil inside the first liquid storage bin 116. For example, the first liquid storage bin 116 and the second liquid storage bin 117 can be stacked, each of the first liquid storage bin 116 and the second liquid storage bin 117 is provided with an opening at their connection, and the openings can be aligned or misaligned by rotating, so that the second liquid storage bin 117 is selectively connected to the first liquid storage bin 116. When the aromatherapy apparatus 100a is in an idle state, the second liquid storage bin 117 can be disconnected from the first liquid storage bin 116, and the second storage bin 117 seals the essential oil inside the first liquid storage bin 116, which can prevent supersaturation and leakage of the essential oil in the liquid guide member 12a and the first liquid storage bin 116 due to long-time communication. When the aromatherapy apparatus 100a is in a working state, the second liquid storage bin 117 can be connected to the first liquid storage bin 116, and essential oil enters the second liquid storage bin 117 from the first liquid storage bin 116 to ensure the supply of essential oil to the liquid guide member 12a, thereby achieving on-demand supply of the essential oil.

In one embodiment, as shown in FIG. 9 and FIG. 10, the aromatherapy apparatus 100a further includes an electronic control component 30 and a shell component 40. The electronic control component 30 may be equivalent to the aforementioned main component 20. The electronic control component 30 is used for controlling the working state of the aromatherapy apparatus 100a, and the shell component 40 is used for mounting the liquid storage component 10a and the electronic control component 30. Specifically, the electronic control component 30 can control the connection state between the heating element 20a and the power source or the heating temperature of the heating element 20a. The volatilization rate of the essential oil is adjusted by changing the temperature of the essential oil heated by the heating element 20a, thereby controlling the working state of the aromatherapy apparatus 100a. For example, as shown in FIG. 9 and FIG. 10, the shell component 40 may include an outer shell 41 and a bottom cover 42, the liquid storage component 10a is mounted at one end of the outer shell 41, and the bottom cover 42 is placed at an end, away from the liquid storage component 10a, of the outer shell 41.

The heating method of the heating element 20a may be resistance heating, electromagnetic heating, infrared heating, or laser heating. The heating element 20a may be separated from the liquid storage component 10a, as shown in FIG. 9 and FIG. 10; or the heating element 20a may be integrated with the liquid storage component 10a, and the heating element 20a is mounted on the liquid storage component 10a, as shown in FIG. 15 and FIG. 16. In one embodiment, as shown in FIG. 9 and FIG. 10, the heating element 20a is arranged on the shell component 40 and faces the liquid guide member 12a, the liquid storage component 10a is detachably connected to the shell component 40, and the heating element 20a is separated from the liquid storage component 10a. Optionally, the heating element 20a is not connected to the liquid storage component 10a, and the two are independently assembled in the shell component 40; and when the essential oil in the liquid storage bin 11 is consumed and the liquid storage component 10a needs to be disassembled and replaced, the heating element 20a is not disassembled with the liquid storage component 10a, and the heating element 20a can continue to be used without replacement. Alternatively, the heating element 20a is detachably connected to the liquid storage component 10a; and when the essential oil in the liquid storage bin 11 is consumed and the liquid storage component 10a needs to be disassembled and replaced, the heating element 20a can be separated from the liquid storage component 10a, and the heating element 20a can be assembled back to the shell component 40 for continued use. The liquid storage component 10a is detachably connected to the shell component 40, and the heating element 20a is separated from the liquid storage component 10a. On the one hand, it is easier to prevent leakage in the liquid storage component 10a. On the other hand, when the liquid storage component 10a is replaced, the heating element 20a does not need to be replaced, so that the heating element 20a can be reused to reduce user's usage cost.

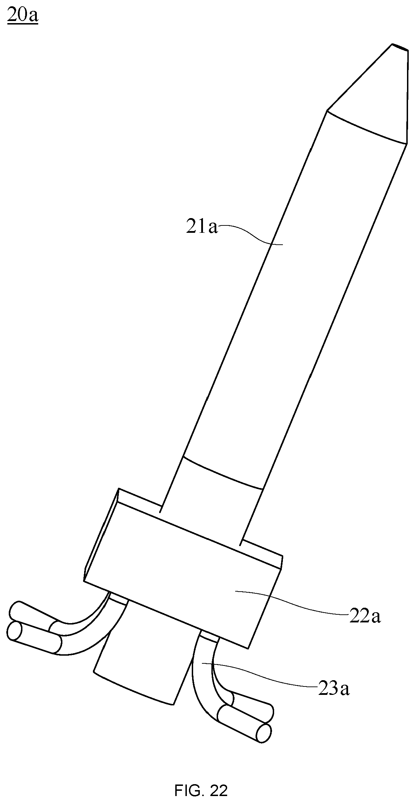

In one embodiment, as shown in FIG. 22, the heating element 20a includes a heating portion 21a, a support portion 22a, and a lead 23a. The heating portion 21a may be cylindrical, the heating portion 21a is connected to one end of the support portion 22a, and the support portion 22a is used for mounting the heating element 20a. The heating portion 21a is electrically connected to the lead 23a, and the heating portion 21a is connected to the power source through the lead 23a.

Optionally, the heating temperature of the essential oil can be controlled below 80°C. If the heating temperature is greater than 80°C, the ingredients in the essential oil may be damaged to some extent, so that the essential oil produces an unpleasant odor at the high temperature. Specifically, the heating temperature of the essential oil may be 80°C, 70°C, 60°C, 50°C, 40°C, 30°C, or the like, which is not limited here.

There are many ways to control the heating temperature of the essential oil in the liquid guide member 12a by the heating element 20a. In one embodiment, as shown in FIG. 9, the electronic control component 30 includes a circuit board 31, a temperature sensor 32, and a power source 33. The power source 33 may be equivalent to the aforementioned battery 23. The power source 33 is electrically connected to the heating element 20a, and the power source 33 provides working electrical energy for the heating element 20a. The temperature sensor 32 is used for monitoring the heating temperature of the heating element 20a. The temperature sensor 32 may be arranged on the heating element 20a or at a position close to the heating element 20a. The temperature sensor 32 transmits the monitored heating temperature to the circuit board 31, and the circuit board 31 can adjust the heating power of the heating element 20a according to the heating temperature monitored by the temperature sensor 32, thereby controlling the heating temperature within a preset range. For example, when the heating temperature is higher than a preset temperature, the heating power of the heating element 20a can be reduced, or the heating element 20a is controlled to stop heating, to lower the heating temperature; and when the heating temperature is lower than the preset temperature, the heating power of the heating element 20a can be increased, or the heating element 20a is controlled to start heating, to increase the heating temperature. By configuring the temperature sensor 32, the temperature sensor 32 works with the circuit board 31, which can control the heating temperature within the preset range, thereby adjusting the volatilization rate of the essential oil.

In one embodiment, the preset distance between the heating part of the heating element 20a and the volatilization surface 122 can be adjusted to adjust the heating temperature of the essential oil in the liquid guide member 12a. For example, the distance between the heating part of the heating element 20a and the volatilization surface 122 can be changed by rotation or push-pull, thereby adjusting the heating temperature of the essential oil in the liquid guide member 12a by the heating element 20a. Specifically, when the heating temperature is higher than the preset temperature, the distance between the heating part and the volatilization surface 122 can be increased by rotation or push-pull to reduce the heat transferred from the heating element 20a to the liquid guide member 12a through radiation, so as to lower the heating temperature; and when the heating temperature is lower than the preset temperature, the distance between the heating part and the volatilization surface 122 can be decreased by rotation or push-pull to increase the heat transferred from the heating element 20a to the liquid guide member 12a through radiation, so as to increase the heating temperature. The heating temperature of the essential oil in the liquid guide member 12a by the heating element 20a is adjusted by changing the distance between the heating part and the volatilization surface 122, so the adjustment operation is simple and reliable.

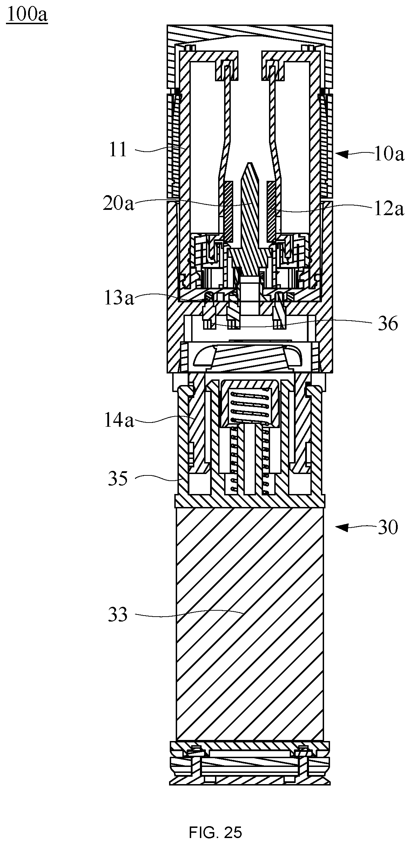

With reference to FIG. 16 and FIG. 25, in one embodiment, the liquid storage component 10a is detachably mounted on the electronic control component 30, the liquid storage component 10a and the electronic control component 30 are provided with a first connector 13a and a second connector 36 respectively, and when the liquid storage component 10a is mounted on the electronic control component 30, the first connector 13a is detachably connected to the second connector 36. By configuring the detachable liquid storage component 10a, the liquid storage component 10a forms a relatively independent module. On the one hand, the essential oil can be sealed inside the liquid storage component 10a to prevent the ingredients in the essential oil from being oxidized. On the other hand, when the essential oil in the liquid storage component 10a is exhausted, the liquid storage component 10a can be replaced for continued use, thereby reducing user's usage cost and improving user experience.

Optionally, the connection method between the first connector 13a and the second connector 36 may be one of adhesion, clamping, or threaded connection. Correspondingly, the first connector 13a may be a double-sided adhesive tape, clamping joint, or bolt, and the second connector 36 may be an adhesive plate, clamping seat, or nut corresponding to the first connector 13a. In one embodiment, the first connector 13a has magnetism, and the first connector 13a is used for magnetic connection with the second connector 36. The first connector 13a has magnetism, so that the first connector 13a can be magnetically connected to the second connector 36. Relatively speaking, the magnetic connection has the advantages of easy disassembly and assembly, secure connection, etc.

When the aromatherapy apparatus 100a is used in a relatively closed environment, for example, the aromatherapy apparatus 100a is placed in a relatively closed box or covers clothes during aromatherapy, the volatilization performance of the aromatherapy apparatus 100a will be affected due to the relatively closed space and poor air circulation. To solve the above problem, in one embodiment, as shown in FIG. 9 and FIG. 10, the aromatherapy apparatus 100a includes a volatilization aid 50, the volatilization aid 50 can produce airflow, and the airflow passes through the liquid guide member 12a and drives the essential oil volatilized from the liquid guide member 12a to diffuse to the external environment. The volatilization aid 50 can accelerate the diffusion rate of the volatilized essential oil to release the essential oil to the external environment, so that the aromatherapy apparatus 100a can also be used normally in a relatively closed environment. The volatilization aid 50 may be equivalent to the aforementioned diffusion aid 21.

Optionally, the volatilization aid 50 may be a fan or air pump. When the fan or air pump is working, strong airflow can be produced, thereby accelerating the diffusion of the essential oil.

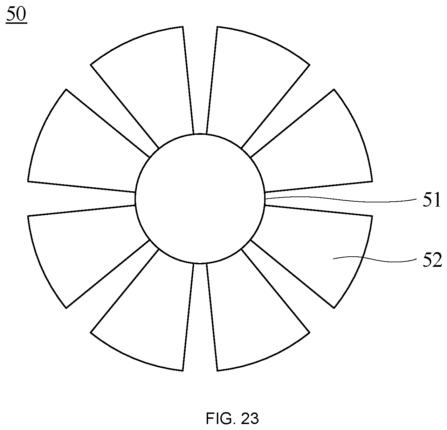

In one embodiment, as shown in FIG. 23, the volatilization aid 50 includes a piezoelectric ceramic vibrator 51 and a plurality of fan blades 52, and the piezoelectric ceramic vibrator 51 is in transmission connection with the fan blades 52. In a working state, the piezoelectric ceramic vibrator 51 vibrates, and the piezoelectric ceramic vibrator 51 drives the fan blades 52 to move to produce airflow, which can accelerate outward flowing of the air in the volatilization tube 112a, thereby driving the volatilized essential oil to diffuse outward, so that the aromatherapy apparatus 100a can also be used normally in a relatively closed environment. The piezoelectric ceramic vibrator 51 has the characteristics of good frequency stability, high accuracy, small size, long lifespan, low noise, etc., which can improve user experience.

With reference to FIG. 15 and FIG. 16, in one embodiment, the aromatherapy apparatus 100a further includes a cover 60, the cover 60 is placed on the liquid storage component 10a to shield the volatilization tube 112a, an air outlet gap is formed between the cover 60 and the liquid storage component 10a, and the air outlet gap is in communication with the volatilization tube 112a. Optionally, the cover 60 includes a first cover 61 and a second cover 62. The first cover 61 is connected to the volatilization hole 1111 of the bin shell 111a, and the second cover 62 is placed outside the first cover 61 and sleeved on an outer side of the bin shell 111a. The essential oil can volatilize and diffuse to the outside of the liquid storage component 10a through the gaps between the volatilization tube 112a, the first cover 61, the second cover 62, and the bin shell 111a. The diffusion path of the essential oil is as shown in FIG. 16. The first cover 61 and the second cover 62 may be integrally formed, or separately machined and then connected and assembled. Relatively speaking, the independent machining of the first cover 61 and the second cover 62 can reduce the difficulty in machining the diffusion shell. Through the cover 60, the essential oil can be effectively diffused in all directions to increase the diffusion rate and prevent external dust from entering the liquid storage component 10a.

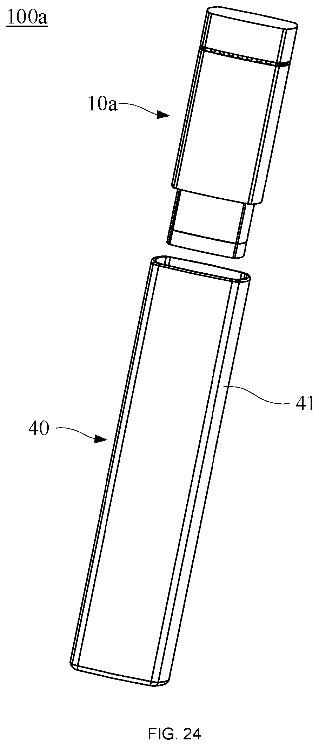

In related technologies, the start method for the aromatherapy apparatus is usually button start, that is, the aromatherapy apparatus is started by pressing a button arranged on an outer surface of the aromatherapy apparatus. With reference to FIGs. 24-26, in one embodiment, the heating element 20a and the liquid storage component 10a are relatively fixed, the liquid storage component 10a has a start position and a closing position relative to the electronic control component 30, and the liquid storage component 10a can switch between the start position and the closing position under external force. The heating element 20a and the liquid storage component 10a are relatively fixed, indicating that the heating element 20a is mounted inside the liquid storage component 10a and the heating element 20a is integrated with liquid storage component 10a; or the heating element 20a is fixedly connected to the liquid storage component 10a through another component (such as a bracket), so that the heating element 20a and the liquid storage component 10a can move synchronously under external force. Optionally, the external force may be torque or tension and pressure, and the external force can change the relative position relationship between the liquid storage component 10a and the electronic control component 30. For example, torque is applied to the liquid storage component 10a or the electronic control component 30, the liquid storage component 10a and the electronic control component 30 rotate relative to each other in a transverse direction, so that the liquid storage component 10a switches between the start position and the closing position; or by the liquid storage component 10a or the electronic control component 30 is pressed or pushed, the liquid storage component 10a and the electronic control component 30 move relative to each other in a longitudinal direction, so that the liquid storage component 10a switches between the start position and the closing position. At the start position, the electronic control component 30 is electrically connected to the heating element 20a, and the essential oil in the liquid storage component 10a is heated, volatilized and diffused to the external environment; and at the closing position, the electronic control component 30 is electrically disconnected from the heating element 20a, so as to start or close the aromatherapy apparatus 100a. The electronic control component 30 is electrically connected to or disconnected from the heating element 20a by switching the position of the liquid storage component 10a under external force, thereby starting or closing the aromatherapy apparatus 100a. Since a button is not required on the outside of the product to control the start of the aromatherapy apparatus 100a, the setting of the start operation does not affect the appearance of the product.

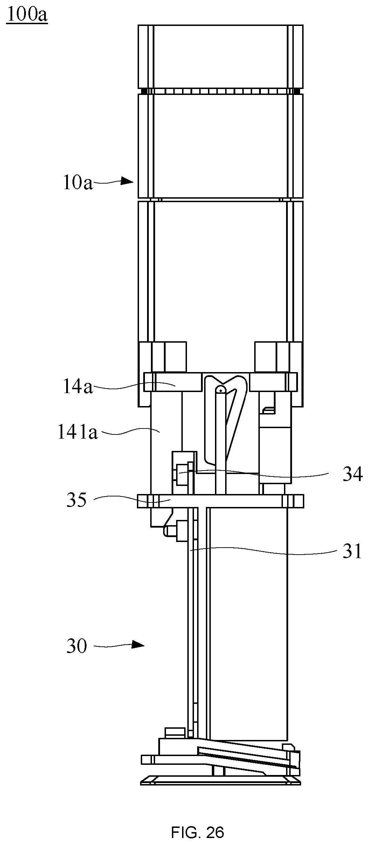

With reference to FIG. 26, in one embodiment, the electronic control component 30 and the liquid storage component 10a are provided with a start switch 34 and a switch trigger 141a respectively. At the start position, the switch trigger 141a triggers to turn on the start switch 34, and the electronic control component 30 is electrically connected to the heating element 20a. At the closing position, the switch trigger 141a turns off the start switch 34, and the electronic control component 30 is disconnected from the heating element 20a. The start switch 34 cooperates with the switch trigger 141a to control the electrical connection or disconnection between the electronic control component 30 and the heating element 20a, which does not affect the appearance of the product and makes the start and closing control operations on the aromatherapy apparatus 100a simple and reliable.

Optionally, the start switch 34 may be one of a button switch, toggle switch, contact switch, or Hall switch. Correspondingly, according to the setting form of the start switch 34, the switch trigger 141a may be rod-like, and the switch trigger 141a can press or toggle the start switch 34; or the switch trigger 141a is provided with a magnetic element to trigger the Hall switch, so as to start or close the aromatherapy apparatus 100a. Optionally, the switch trigger 141a is connected to the liquid storage bin 11 or another component of the liquid storage component 10a, so that the switch trigger 141a can move synchronously with the liquid storage component 10a. When the liquid storage component 10a switches between the start position and the closing position, the start switch 34 cooperates with the switch trigger 141a to control the electrical connection or disconnection between the electronic control component 30 and the heating element 20a.

With reference to FIG. 25 and FIG. 26, in one embodiment, the liquid storage component 10a includes a first bracket 14a, the electronic control component 30 includes a second bracket 35, and the first bracket 14a is connected to the second bracket 35 in a sliding manner. Under external force, the first bracket 14a slides relative to the second bracket 35, so that the liquid storage component 10a switches between the start position and the closing position. It should be noted that the positions of the first bracket 14a and the second bracket 35 may be interchanged, that is, the first bracket 14a may be arranged on a side where the electronic control component 30 is located, and the second bracket 35 may be arranged on a side where the liquid storage component 10a is located. The position of the liquid storage component 10a is switched by the relative sliding of the first bracket 14a and the second bracket 35 to start or close the aromatherapy apparatus 100a, so that the setting of the start operation does not affect the appearance of the product.

Described above are only some embodiments of the present application, and the scope of protection of the present application is not limited thereto. Any equivalent apparatus or equivalent process transformation made using the description and accompanying drawings of the present application, directly or indirectly applied in other related technical fields, is also included in the scope of patent protection of the present application.

## Claims

1. A fragrance apparatus, comprising:
a fragrance component and a main component, the fragrance component comprising a liquid storage bin, a liquid guide, a heater, and a first connector, wherein the first connector can be detachably connected to the main component;
the liquid storage bin is provided with a closed liquid storage chamber for storing an essential oil, the liquid guide is in fluid communication with the liquid storage chamber, the liquid guide is used for transporting the essential oil in the liquid storage chamber, and the heater can heat the essential oil in the liquid guide to volatilize and diffuse the essential oil outward; and
under external force, the relative movement of the fragrance component and the main component triggers opening or closing of the fragrance apparatus.

2. The fragrance apparatus according to claim 1, wherein the main component is provided with a second connector, and the second connector is detachably connected to the first connector.

3. The fragrance apparatus according to claim 2, wherein the connection method between the second connector and the first connector is one of adhesion, clamping, threaded connection, or magnetic connection.

4. The fragrance apparatus according to claim 1, wherein the fragrance component has a start position and a closing position relative to the main component; under the external force, the fragrance component moves relative to the main component, and the fragrance component can switch between the start position and the closing position, wherein
at the start position, the main component is electrically connected to the heater, and the fragrance apparatus is started; at the closing position, the main component is electrically disconnected from the heater, and the fragrance apparatus is closed.

5. The fragrance apparatus according to claim 4, wherein the main component and the liquid storage component are provided with a start switch and a switch trigger respectively; at the start position, the switch trigger triggers to turn on the start switch, and the main component is electrically connected to the heater; at the closing position, the switch trigger turns off the start switch, and the main component is electrically disconnected from the heater.

6. The fragrance apparatus according to claim 4, wherein the fragrance component comprises a first bracket, the main component comprises a second bracket, and the first bracket is connected to the second bracket in a sliding manner; and
under the external force, the first bracket slides relative to the second bracket, and the fragrance component switches between the start position and the closing position to trigger the opening or closing of the fragrance apparatus.

7. The fragrance apparatus according to claim 1, wherein the liquid storage bin comprises a bin shell and a bracket, the bin shell is of a cylindrical structure with an open end, the bracket is connected to the open end of the bin shell, and the bracket is accommodated inside the bin shell; and
the bracket is provided with an oil supply hole, the liquid guide is mounted on the bracket, the liquid guide is at least partially blocked at the oil supply hole, and the liquid storage chamber is enclosed by the bin shell, the bracket, and the liquid guide.

8. The fragrance apparatus according to claim 7, wherein the fragrance component comprises a base, the base is connected to the open end of the bin shell, the bracket is partially accommodated in the base, and the liquid guide is mounted inside a volatilization chamber enclosed by the base and the bracket; and
a bottom of the base is provided with an air inlet, a side wall of the base is provided with a volatilization hole, both the air inlet and the volatilization hole are in communication with the volatilization chamber, and the essential oil volatilized from the liquid guide can be released and diffused to the outside of the fragrance component via the volatilization hole.

9. The fragrance apparatus according to claim 8, wherein the fragrance component comprises a liquid absorber, the liquid absorber is mounted on the base and accommodated in the volatilization chamber, the liquid absorber is spaced apart from the liquid guide, and the liquid absorber can adsorb the essential oil leaking from the liquid guide.

10. The fragrance apparatus according to claim 1, wherein the main component comprises a diffusion aid, the diffusion aid is mounted at an end, close to the liquid guide, of the main component, the diffusion aid can produce an airflow, and the airflow passes through the liquid guide and drives the essential oil volatilized from the liquid guide to diffuse outward.

11. The fragrance apparatus according to claim 1, wherein the heater is spaced apart from the liquid guide.

12. The fragrance apparatus according to claim 11, wherein the liquid guide has a liquid guide surface and a volatilization surface, the liquid guide surface is in fluid communication with the liquid storage chamber, the volatilization surface faces a heating part of the heater at a preset distance, and the essential oil entering the liquid guide from the liquid guide surface is heated on the volatilization surface and volatilized to an external environment.

13. The fragrance apparatus according to claim 12, wherein the liquid guide is made of a porous material, a capillary microporous structure is formed between the liquid guide surface and the volatilization surface, and the essential oil is transported by the capillary microporous structure.

14. The fragrance apparatus according to claim 12, wherein the preset distance between the heating part and the volatilization surface can be adjusted to regulate the heating temperature of the essential oil in the liquid guide.

15. The fragrance apparatus according to claim 12, wherein the liquid storage bin is provided with an oil outlet through hole, and the liquid guide is connected to the liquid storage bin, wherein the liquid guide surface faces the oil outlet through hole to transfer the essential oil from the oil outlet through hole to the volatilization surface.

16. The fragrance apparatus according to claim 15, wherein the fragrance component is provided with a pressure balancing structure, the pressure balancing structure balances the pressure inside the liquid storage chamber to maintain consistency with the external environment, and in at least one conventional working posture, the position of the oil outlet through hole is lower than an air inlet of the pressure balancing structure.

17. The fragrance apparatus according to claim 16, wherein the pressure balancing structure comprises an air inlet passage, the air inlet passage is connected to the liquid storage chamber and the external environment, the air inlet is arranged at the connection between the air inlet passage and the liquid storage chamber, and a control valve is provided at the air inlet for ventilation and prevention of essential oil leakage.

18. The fragrance apparatus according to claim 12, wherein the liquid storage bin comprises a bin shell and a volatilization tube, the volatilization tube is mounted inside the bin shell, the volatilization tube is hollow, and the liquid guide is accommodated inside a hollow cavity of the volatilization tube; and
the heating part of the heater is accommodated inside the volatilization tube, and the heater is spaced apart from an inner wall of the liquid guide.

19. The fragrance apparatus according to claim 1, wherein the main component comprises a circuit board, a temperature sensor, and a power source, the power source is electrically connected to the heater, the temperature sensor is used for monitoring the heating temperature of the heater and the circuit board can adjust the heating power of the heater according to the heating temperature monitored by the temperature sensor.

20. The fragrance apparatus according to claim 12, wherein the liquid guide comprises a sealing layer, the sealing layer is attached to a peripheral surface of the liquid guide surface, the sealing layer is provided with a liquid guide hole, and the liquid guide hole is in fluid communication with the liquid storage chamber.
